# EUROPEAN PATENT APPLICATION

(11) **EP 4 835 072 A1**
(43) Date of publication of application: **04.11.2026**
(21) Application number: 23963127.8
(22) Date of filing: 27.12.2023
(51) Int. Cl.: C12N 15/11, C12N 5/10, C12N 15/09, C12P 21/00

(54) **NOVEL PROMOTER**

(71) Applicant: CHUGAI SEIYAKU KABUSHIKI KAISHA, Tokyo 115-8543 (JP)
(72) Inventor: BETANCUR MEDINA, Juan Guillermo, Tokyo 115-8543 (JP)
(74) Representative: Vossius & Partner Patentanwälte Rechtsanwälte mbB
(86) International application number: PCT/JP2023/046973
(87) International publication number: WO 2025/141778

(57) **Abstract**

Provided is a novel promoter for expressing a recombinant protein with a mammalian cell as a host.

Exemplified is a promoter consisting of a polynucleotide in which a partial region of a CAG promoter sequence is deleted, wherein, in a CAG promoter of SEQ ID NO: 1, nucleic acids are deleted in one or both of the region (a) of positions 469 to 571 and the region (b) of positions 799 to 1563.

## Description

### TECHNICAL FIELD

The present invention relates to a promoter for expressing a recombinant protein with a mammalian cell as a host. The present invention also relates to a method for producing a recombinant protein using the promoter.

### BACKGROUND ART

In order to express a recombinant protein with a mammalian cell as a host, it is widely practiced to introduce an expression vector carrying a gene for a target protein into a host cell and then culture the cell. The protein expression vector comprises sequences necessary for transcription and translation of the gene, such as a promoter, terminator, and translation initiation signal. The production of a recombinant protein involves various factors, such as the sequences of these regions within the expression vector and the culture conditions of the recombinant cells. In particular, the selection of the promoter is important, as it affects the expression level of the recombinant protein. Several plasmid vectors and viral vectors employing the CAG promoter, a strong promoter, are commercially available.

The CAG promoter is constructed as a hybrid promoter in which a CMV enhancer is bound to a modified chicken β-actin promoter. The wild-type CAG promoter is described in Gene 108:193-199, "Efficient Selection for High-Expression Transfectants with a Novel Eukaryotic Vector" (NPL 1). The matters described in this literature are incorporated herein by reference. The CAG promoter is also described in Japanese Patent Laid-Open No. H3-168087.

The DNA sequence of the wild-type CAG promoter is shown in SEQ ID NO: 1.

Japanese Patent No. 5670330 (WO2010/015079) discloses a promoter improved from the CAG promoter.

### CITATION LIST

### PATENT LITERATURE

PTL 1: Japanese Patent No. 5670330 (WO2010/015079)
PTL 2: Japanese Patent Laid-Open No. H3-168087

### NON PATENT LITERATURE

NPL 1: Niwa et al., 1991, Gene 108:193-199

### SUMMARY OF INVENTION

### TECHNICAL PROBLEM

An object of the present invention is to provide a highly versatile promoter that is superior in various respects to the CAG promoter, an existing high-expression promoter, for the production of recombinant proteins using mammalian cells.

### SOLUTION TO PROBLEM

The present inventors have conducted studies to develop a promoter superior to the conventional CAG promoter. The present inventors have then identified a deletable site within the CAG promoter and, by removing this site, have found a novel modified CAG promoter that is easy to use and exhibits high expression levels. This promoter is referred to as the "promoter of the present invention."

The promoter of the present invention comprises a promoter in which nucleic acids are deleted in one or both of the following regions in a CAG promoter described in SEQ ID NO:1:
(a) 469 to 571;
(b) 799 to 1563.

The invention disclosed in the present application includes the following aspects.
(1) An isolated polynucleotide consisting of a nucleotide sequence in which at least the region from base 469 to base 571 is deleted from a nucleotide sequence described in SEQ ID NO: 1, or consisting of a nucleotide sequence having 90% or more identity thereto.
(2) The polynucleotide according to the above (1), consisting of a nucleotide sequence described in SEQ ID NO: 2, or consisting of a nucleotide sequence having 90% or more identity thereto.
(3) An isolated polynucleotide consisting of a nucleotide sequence in which at least the region from base 799 to base 1563 is deleted from the sequence described in SEQ ID NO: 1, or consisting of a nucleotide sequence having 90% or more identity thereto.
(4) The polynucleotide according to the above (3), consisting of a nucleotide sequence described in SEQ ID NO: 3, or consisting of a nucleotide sequence having 90% or more identity thereto.
(5) An isolated polynucleotide consisting of a nucleotide sequence in which at least the following regions (a) and (b) are deleted from a nucleotide sequence described in SEQ ID NO: 1, or consisting of a nucleotide sequence having 90% or more identity thereto:
   (a) the region from base 469 to base 571; and
   (b) the region from base 799 to base 1563.
(6) The polynucleotide according to the above (5), consisting of the sequence described in SEQ ID NO: 4, or consisting of a nucleotide sequence having 90% or more identity thereto.
(7) A promoter consisting of the polynucleotide according to any one of the above (1) to (6).
(8) A vector comprising the promoter according to the above (7).
(9) A vector comprising the promoter according to the above (7) and a gene encoding a protein.
(10) A transformed cell obtained by transforming a mammalian cell with the vector according to the above (9).
(11) The transformed cell according to the above (10), wherein the mammalian cell is a CHO cell.
(12) A method for producing a recombinant protein, comprising a step of culturing the transformed cell according to the above (11) to produce a recombinant protein, and a step of recovering the produced recombinant protein from the resulting culture.
(13) The method according to the above (12), which is a method for producing an antibody, wherein the vector comprises a promoter consisting of the sequence described in any one of SEQ ID NOs: 2, 3, or 4, and a gene encoding an amino acid sequence comprising a VL or a VH of the antibody, or encoding amino acid sequences comprising the VL and VH of the antibody.
(14) An isolated polynucleotide fragment having a promoter function, consisting of a sequence having at least 70%, at least 80%, at least 90%, or 100% identity to the sequence described in any one of SEQ ID NOs: 2, 3, or 4.
(15) The vector according to the above (9), wherein the protein is a heavy chain (H chain) or a light chain (L chain) of an antibody.
(16) A cell comprising a gene into which a promoter consisting of a nucleotide sequence described in any one of SEQ ID NOs: 2, 3, or 4 is incorporated.
(17) The cell according to the above (16), wherein the gene encodes an antibody polypeptide.
(18) A method for producing an antibody using a cell comprising a gene into which a promoter consisting of a nucleotide sequence described in any one of SEQ ID NOs: 2, 3, or 4 is incorporated.

### ADVANTAGEOUS EFFECTS OF INVENTION

The present inventors have identified the minimum required sequence of the CAG promoter and developed a promoter that is excellent in terms of expression levels of exogenous genes and handling. Similar to the wild-type CAG promoter, the promoter of the present invention has a wide host range and allows to establish a cell line that stably expresses exogenous genes using various types of cells derived from various animals. Furthermore, the promoter of the present invention is more readily amplified and maintained than the wild-type CAG promoter. For example, the promoter of the present invention can be readily amplified by PCR, since the GC-rich region 469 to 571 has been deleted from the DNA sequence of the wild-type CAG promoter (SEQ ID NO: 1). Since the DNA fragment comprising the promoter of the present invention can be amplified by PCR, it is possible to avoid the need for subcloning using an intermediate plasmid. Therefore, by using the promoter of the present invention, it is possible to readily construct an expression vector into which an exogenous gene has been introduced.

The promoter of the present invention can be used in the production of antibody pharmaceuticals produced with animal cells constructed using genetic recombination techniques as cell substrates for production. In addition, the present invention is highly versatile and can serve as a platform technology for the production of not only antibodies but also various protein pharmaceuticals.

### BRIEF DESCRIPTION OF DRAWINGS

Fig. 1 shows a circular DNA comprising the sequence of the promoter of the present invention represented by SEQ ID NO: 2.
Fig. 2 shows a circular DNA comprising the sequence of the promoter of the present invention represented by SEQ ID NO: 3.
Fig. 3 shows the expression of luciferase driven by the promoter of the present invention and the wild-type CAG promoter.
Fig. 4 shows the amplification of promoter fragments by PCR.

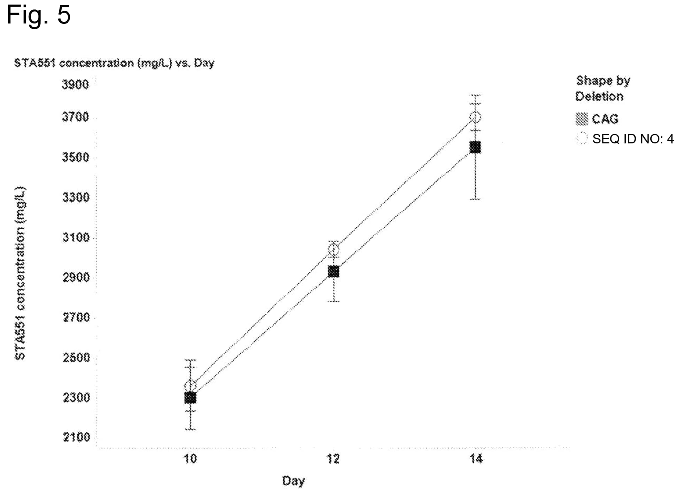

Fig. 5 shows the expression of monoclonal antibodies driven by the promoter of the present invention and the wild-type CAG promoter.

### DESCRIPTION OF EMBODIMENTS

Unless otherwise defined, all technical and scientific terms used herein shall have the same meanings as generally understood by those ordinarily skilled in the art to which the present invention pertains. Although any methods and materials similar or equivalent to those described herein can be used in the practice or testing of the present invention, suitable methods and materials are shown herein. All publications mentioned below are incorporated herein by reference.

The promoter of the present invention is a promoter in which a portion of the existing CAG promoter is deleted, which makes it easy to use and improves the expression levels of exogenous genes in mammalian cells. Specifically, it is a promoter in which nucleic acids are deleted in one or more of the following regions (a) and (b) in a CAG promoter sequence described in SEQ ID NO:1:
(a) From the T at position 469 to the C at position 571
(b) From the G at position 799 to the C at position 1563

As a specific example of the promoter of the present invention, a promoter consisting of the sequence described in SEQ ID NO: 2, in which the nucleotides from positions 469 to 571 of the nucleotide sequence described in SEQ ID NO: 1 are deleted, can be mentioned.

As a specific example of the promoter of the present invention, a promoter consisting of the sequence described in SEQ ID NO: 3, in which the nucleotides from positions 799 to 1563 of the nucleotide sequence described in SEQ ID NO: 1 are deleted, can be mentioned.

As a specific example of the promoter of the present invention, a promoter consisting of the sequence described in SEQ ID NO: 4, in which the nucleotides from positions 469 to 571 and 799 to 1563 of the nucleotide sequence described in SEQ ID NO: 1 are deleted, can be mentioned.

SEQ ID NOs: 2 and 3 were prepared by deleting a partial sequence of the CAG promoter in a circular DNA (plasmid) in which the CAG promoter of SEQ ID NO: 1 had been cloned. SEQ ID NO: 4 of the present invention was prepared by artificial synthesis. Details regarding the preparation of the promoter of the present invention will be described later.

The promoter of the present invention can be readily amplified by PCR since the polynucleotide fragment, which is DNA, lacks the GC-rich region 469 to 571. In contrast, the wild-type CAG promoter comprises a GC-rich region and therefore cannot be amplified by PCR as is.

The promoter of the present invention can be maintained and stored as such as a polynucleotide fragment or by cloning it into a circular or linear plasmid. Alternatively, the promoter of the present invention may be inserted into an appropriate vector for cloning, and maintained and stored with the vector incorporated in a microorganism such as Escherichia coli.

To produce a recombinant protein using the promoter of the present invention, it is preferable to use an expression vector comprising the promoter of the present invention and a polynucleotide encoding the recombinant protein. When producing a recombinant antibody protein with a mammalian cell as a host, a plasmid vector is generally used as the expression vector.

However, any vector to which the promoter of the present invention can be applied may be used, as long as it can express the target protein in animal cells, cell-free protein translation systems, or the like, and the type of vector is not limited.

The expression vector can further comprise components that allow stable expression of the protein when incorporated into mammalian cells. The expression vector preferably has an appropriate restriction enzyme site downstream of the promoter of the present invention, into which a gene encoding the target protein can be incorporated. It is preferable to also incorporate into the expression vector a marker gene for selecting or visualizing cells that have been transformed by incorporating a protein expression vector, and a separate promoter for driving the expression of the marker gene is positioned upstream thereof. The separate promoter is generally a promoter other than the promoter of the present invention. The expression vector may further comprise components for maintaining the vector in Escherichia coli, in order to facilitate cloning in Escherichia coli.

There is no limitation on the recombinant protein to be expressed using the promoter of the present invention. Examples of proteins for pharmaceuticals include antibody proteins (immunoglobulins), various enzymes, and human-derived receptor proteins.

In one aspect, the promoter of the present invention is used in the production of antibody pharmaceuticals. In the process of producing antibody pharmaceuticals produced using genetic recombination techniques, a gene for the target antibody is inserted into a vector suitable for recombinant protein expression to construct a gene expression construct (expression vector). Next, the expression vector is introduced (transfected) into a host cell to obtain an antibody-producing cell.

The antibody gene can comprise a nucleic acid encoding an amino acid sequence comprising a VL (e.g., light chain amino acid sequence) and an amino acid sequence comprising a VH (e.g., heavy chain amino acid sequence). When expressing a recombinant antibody protein, a nucleic acid encoding an amino acid sequence comprising a VL and a nucleic acid encoding an amino acid sequence comprising a VH are each positioned downstream of the promoter of the present invention. The nucleic acid encoding an amino acid sequence comprising a VL and the nucleic acid encoding an amino acid sequence comprising a VH may be comprised in the same expression vector. The nucleic acid encoding an amino acid sequence comprising a VL and the nucleic acid encoding an amino acid sequence comprising a VH may be comprised in separate expression vectors.

There is no limitation on the mammalian cell used to express a recombinant protein using the promoter of the present invention. Examples thereof include Chinese hamster ovary cells (CHO cells) (K1 strain, DG44 strain, and DXB11 strain), human embryonic kidney cells (HEK cells), human leukemia cell-derived cells (HL-60 cells), human cervical cancer-derived cells (HeLa cells), human leukemia T-cell-derived cells (Jurkat), African green monkey kidney cell-derived cells (COS cells), mouse myeloma cells such as Sp2/0 cells and NS0 cells, and induced pluripotent stem cells (iPSCs).

Recombinant proteins produced using CHO cells have been confirmed to be safe for use as pharmaceuticals and are now a standard method. Therefore, one aspect of producing a protein as an active ingredient of a pharmaceutical using the promoter of the present invention is a recombinant protein expression system using CHO cells. The promoter of the present invention can be suitably used for the construction of a CHO cell line that highly expresses antibodies.

Means for delivering genes into cells are well known in the art, and may be appropriately selected depending on the cell used as a host. Commercially available gene delivery systems may be used to transform cells with an expression vector comprising the promoter of the present invention. Examples of methods for introducing an expression vector into mammalian cells include electroporation, a method using reagents for transfection such as lipofection, and a method using a viral vector. Examples of methods for inserting an exogenous gene into the host genome of mammalian cells include random integration, targeted integration (site-specific gene insertion using a recombinase, which is a sequence-specific recombinant enzyme), and methods using transposon vectors and site-specific nucleases. The method of site-specific gene introduction into a specific locus of the host genome is showing promise as a method for efficiently obtaining cells with excellent production levels of the target protein and passage stability.

When developing a recombinant protein as a pharmaceutical, a procedure is performed which includes culturing microorganisms or cells into which the gene for a target protein has been introduced, extracting, purifying, concentrating the target protein, and formulating.

Mammalian cells are transformed with an expression vector comprising the promoter of the present invention and a gene for a target protein to obtain a transformant (transformed cell) capable of producing a recombinant protein, the resulting transformant is cultured, and the produced recombinant protein is recovered from the resulting culture, which allows to highly efficiently produce the recombinant protein.

In recovering the recombinant protein from the culture of the transformant, purification procedures using chromatography, such as affinity chromatography, ionexchange chromatography, hydrophobic interaction chromatography, and gel filtration chromatography, may be used in combination, whereby the recombinant protein can be recovered with high efficiency and high purity.

In an additional aspect of the present invention is proposed a purified or isolated polynucleotide fragment (DNA) having a promoter function, the polynucleotide fragment (DNA) consisting of a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more identity to the nucleotide sequence described in SEQ ID NO: 4. Here, when the expression induced by a promoter consisting of these DNAs (i.e., having 70% or more identity to the nucleotide sequence described in SEQ ID NO:4) shows an expression at a level equivalent to or higher than, specifically 60%, 65%, 70%, 75%, 80%, 85%, 90%, 95%, 96%, 97%, 98%, 99% or more, the expression induced by the wild-type CAG promoter consisting of the DNA described in SEQ ID NO: 1, the promoter is said to "have a promoter function." The efficiency of expression mentioned herein refers to the efficiency of expression when this promoter and the promoter described in SEQ ID NO: 1 are each bound to a substantially identical reporter gene, transfected into a substantially identical cell line, and cultured under the same conditions. It typically refers to the efficiency of expression when cultured under conditions optimal for the expression of the reporter gene by the promoter.

### EXAMPLES

Hereinafter, the present invention will be described in more detail by way of Examples. It should be noted that these Examples are for the purpose of describing the present invention and are not intended to limit the scope of the present invention.

### [Preparation of Promoter of Present Invention]

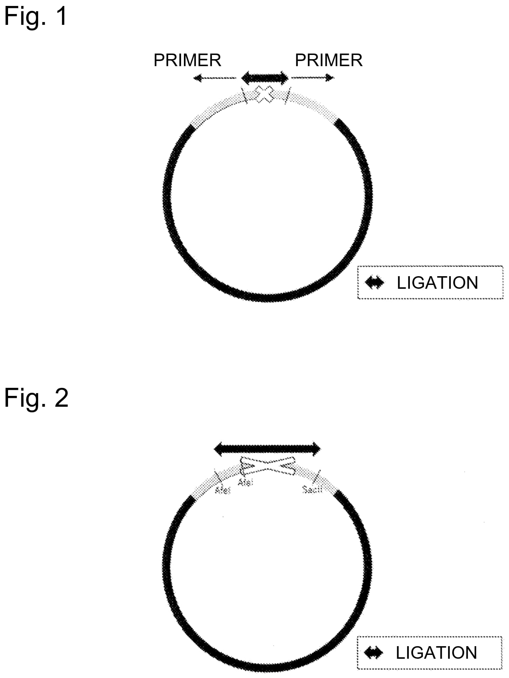

A circular DNA comprising the sequence of the promoter of the present invention represented by SEQ ID NO: 2 is shown in Fig. 1. A circular DNA comprising the promoter of the present invention was prepared by performing inverse PCR using the primer CAGDel3F (AGCCAATCAGAGCGGCGC: SEQ ID NO: 5) and the primer CAGDel3R2 (ATTAAAAAATAAATAAATACAAAATTGGGGGTGG: SEQ ID NO: 6) to delete 103 base pairs from positions 469 to 571, followed by ligation using T4 ligase.

A circular DNA comprising the sequence of the promoter of the present invention represented by SEQ ID NO: 3 is shown in Fig. 2. A circular DNA comprising the promoter of the present invention was prepared by removing 765 base pairs from positions 799 to 1563 using the restriction enzymes AfeI and SacII, followed by ligation using T4 ligase.

The promoter of the present invention represented by SEQ ID NO: 4 was prepared by artificially synthesizing a DNA fragment consisting of a sequence in which the base pairs from positions 469 to 571 and 799 to 1563 were removed according to the method described above.

### [Example 1: Luciferase Expression]

A luciferase reporter gene was introduced downstream of the promoter in which nucleic acids from positions 469 to 571 of SEQ ID NO: 1 are deleted (i.e., the promoter of SEQ ID NO: 2), the promoter in which nucleic acids from positions 799 to 1563 are deleted (i.e., the promoter of SEQ ID NO: 3), or the wild-type CAG promoter. A plasmid was transfected into CHO cells by a site-specific gene insertion method (Targeted Integration system) using a recombinase, and after selecting a stable expression cell pool, luciferase expression was evaluated in a fed-batch culture for 14 days (n = 3). Luciferase expression was measured on days 3, 5, 7, 10, 12, and 14, and normalized to luciferase expressed under the control of the wild-type CAG promoter.

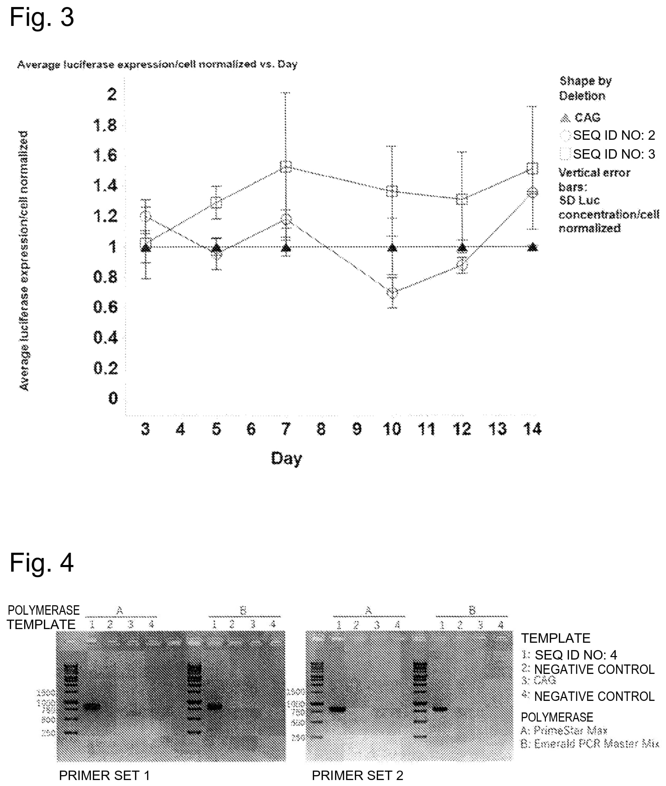

The results are shown in Fig. 3. The promoter of SEQ ID NO: 2 did not affect reporter gene expression compared to the wild-type CAG. On the other hand, the promoter of SEQ ID NO: 3 showed about 40% higher reporter gene expression from days 7 to 14 compared to the wild-type CAG.

### [Example 2: PCR Amplification of Deleted Promoter Fragment]

The promoter of SEQ ID NO: 4 was amplified by PCR using two different polymerases and primer sets.

The results are shown in Fig. 4. When using the promoter of SEQ ID NO: 4 as a template, optimization of the PCR reaction was not required, and the target PCR product (about 860 bp) was obtained under all conditions. On the other hand, with the wild-type CAG, the target product (about 1,730 bp) could not be amplified under any conditions. These results confirmed that the promoter of SEQ ID NO: 4 can be amplified by PCR.

### [Example 3: Expression of STA551]

STA551 is a switch antibody applying Switch-Ig (registered trademark) developed by CHUGAI PHARMACEUTICAL CO., LTD. It is activated by recognizing adenosine triphosphate (ATP), which is said to be present at high concentrations in tumor tissues, as a switch molecule, and binds to the target antigen CD137.

Plasmids with four copies of the L chain and two copies of the H chain of STA551 using SEQ ID NO: 4 or wild-type CAG as the promoter were prepared.

The wild-type CAG plasmid was prepared in two steps. First, to introduce ligation/homology sites, the L/H genes were each subcloned into intermediate plasmids comprising appropriate homology sites for linkage to wild-type CAG, using a general cloning method. Next, after amplifying and purifying each intermediate plasmid, all fragments were assembled into a single expression plasmid using a homology/ligation-based method. This process requires two Escherichia coli transformation steps and takes at least five days.

On the other hand, the plasmid of the promoter of SEQ ID NO: 4 was prepared in a single step without the need for intermediate plasmids, since the fragment comprising SEQ ID NO: 4 can be amplified by PCR. The DNA fragments comprising the promoter of SEQ ID NO: 4, the L/H genes, and homology sites for ligation were prepared by PCR using appropriate primers. The assembly of the plasmid comprising the promoter of SEQ ID NO: 4 requires only one Escherichia coli transformation step and can be done in just three days.

The plasmid was transfected into CHO cells by a site-specific gene insertion method (Targeted Integration system) using a recombinase, and after selecting a stable expression cell pool, STA551 expression was evaluated on days 10, 12, and 14 in a fed-batch culture for 14 days (n = 3). STA551 expression was comparable between the two promoters.

These results confirmed that the use of the promoter of SEQ ID NO: 4 facilitates the assembly of complex plasmids, and that the promoter of SEQ ID NO: 4 can be used for monoclonal antibody expression at a level at least equivalent to that of wild-type CAG.

While the suitable aspects of the present invention have been shown above, it should be recognized and understood that various modifications can be made thereto. The appended claims are intended to encompass all such modifications without departing from the spirit and scope of the present invention.

The promoter sequence described in the present invention is shown.
SEQ ID NO:1_CAG promoter 
GTCGACATTGATTATTGACTAGTTATTAATAGTAATCAATTACGGGGTCATTAGT
TCATA
GCCCATATATGGAGTTCCGCGTTACATAACTTACGGTAAATGGCCCGCCTGGCTG
ACCGC
CCAACGACCCCCGCCCATTGACGTCAATAATGACGTATGTTCCCATAGTAACGCC
AATAG
GGACTTTCCATTGACGTCAATGGGTGGAGTATTTACGGTAAACTGCCCACTTGGC
 
AGTAC

ATCAAGTGTATCATATGCCAAGTACGCCCCCTATTGACGTCAATGACGGTAAATG

GCCCG

CCTGGCATTATGCCCAGTACATGACCTTATGGGACTTTCCTACTTGGCAGTACAT

CTACG

TATTAGTCATCGCTATTACCATGGTCGAGGTGAGCCCCACGTTCTGCTTCACTCTC

CCCA

TCTCCCCCCCCTCCCCACCCCCAATTTTGTATTTATTTATTTTTTAATTATTTTGTG

CAG

CGATGGGGGCGGGGGGGGGGGGGGCGCGCGCCAGGCGGGGCGGGGCGGGGC

GAGGGGC

GGGGCGGGGCGAGGCGGAGAGGTGCGGCGGCAGCCAATCAGAGCGGCGCGCTC

CGAAAGT

TTCCTTTTATGGCGAGGCGGCGGCGGCGGCGGCCCTATAAAAAGCGAAGCGCGC

GGCGGG

CGGGAGTCGCTGCGCGCTGCCTTCGCCCCGTGCCCCGCTCCGCCGCCGCCTCGCG

CCGCC

CGCCCCGGCTCTGACTGACCGCGTTACTCCCACAGGTGAGCGGGCGGGACGGCC

CTTCTC

CTCCGGGCTGTAATTAGCGCTTGGTTTAATGACGGCTTGTTTCTTTTCTGTGGCTG

CGTG

AAAGCCTTGAGGGGCTCCGGGAGGGCCCTTTGTGCGGGGGGAGCGGCTCGGGGG

GTGCGT

GCGTGTGTGTGTGCGTGGGGAGCGCCGCGTGCGGCTCCGCGCTGCCCGGCGGCT

GTGAGC

GCTGCGGGCGCGGCGCGGGGCTTTGTGCGCTCCGCAGTGTGCGCGAGGGGAGCG

CGGCCG

GGGGCGGTGCCCCGCGGTGCGGGGGGGGCTGCGAGGGGAACAAAGGCTGCGTG
 
CGGGGTG
TGTGCGTGGGGGGTGAGCAGGGGGTGTGGGCGCGTCGGTCGGGCTGCAACCCC
CCCTGC
ACCCCCCTCCCCGAGTTGCTGAGCACGGCCCGGCTTCGGGTGCGGGGCTCCGTAC
GGGGC
GTGGCGCGGGGCTCGCCGTGCCGGGCGGGGGGTGGCGGCAGGTGGGGGTGCCG
GGCGGGG
CGGGGCCGCCTCGGGCCGGGGAGGGCTCGGGGGAGGGGCGCGGCGGCCCCCGG
AGCGCCG
GCGGCTGTCGAGGCGCGGCGAGCCGCAGCCATTGCCTTTTATGGTAATCGTGCGA
GAGGG
CGCAGGGACTTCCTTTGTCCCAAATCTGTGCGGAGCCGAAATCTGGGAGGCGCC
GCCGCA
CCCCCTCTAGCGGGCGCGGGGCGAAGCGGTGCGGCGCCGGCAGGAAGGAAATG
GGCGGGG
AGGGCCTTCGTGCGTCGCCGCGCCGCCGTCCCCTTCTCCCTCTCCAGCCTCGGGG
CTGTC
CGCGGGGGGACGGCTGCCTTCGGGGGGGACGGGGCAGGGCGGGGTTCGGCTTCT
GGCGTG
TGACCGGCGGCTCTAGAGCCTCTGCTAACCATGTTCATGCCTTCTTCTTTTTCCTA
CAGC
TCCTGGGCAACGTGCTGGTTATTGTGCTGTCTCATCATTTTGGCAAAccggt
SEQ ID NO:2_469~571(Del3) 
GTCGACATTGATTATTGACTAGTTATTAATAGTAATCAATTACGGGGTCATTAGT
TCATA
GCCCATATATGGAGTTCCGCGTTACATAACTTACGGTAAATGGCCCGCCTGGCTG
ACCGC
CCAACGACCCCCGCCCATTGACGTCAATAATGACGTATGTTCCCATAGTAACGCC
 
AATAG
GGACTTTCCATTGACGTCAATGGGTGGAGTATTTACGGTAAACTGCCCACTTGGC
AGTAC
ATCAAGTGTATCATATGCCAAGTACGCCCCCTATTGACGTCAATGACGGTAAATG
GCCCG
CCTGGCATTATGCCCAGTACATGACCTTATGGGACTTTCCTACTTGGCAGTACAT
CTACG
TATTAGTCATCGCTATTACCATGGTCGAGGTGAGCCCCACGTTCTGCTTCACTCTC
CCCA
TCTCCCCCCCCTCCCCACCCCCAATTTTGTATTTATTTATTTTTTAATAGCCAATCA
GAG
CGGCGCGCTCCGAAAGTTTCCTTTTATGGCGAGGCGGCGGCGGCGGCCCTAT
AAAAA
GCGAAGCGCGCGGCGGGCGGGAGTCGCTGCGCGCTGCCTTCGCCCCGTGCCCCG
CTCCGC
CGCCGCCTCGCGCCGCCCGCCCCGGCTCTGACTGACCGCGTTACTCCCACAGGTG
AGCGG
GCGGGACGGCCCTTCTCCTCCGGGCTGTAATTAGCGCTTGGTTTAATGACGGCTT
GTTTC
TTTTCTGTGGCTGCGTGAAAGCCTTGAGGGGCTCCGGGAGGGCCCTTTGTGCGGG
GGGAG
CGGCTCGGGGGGTGCGTGCGTGTGTGTGTGCGTGGGGAGCGCCGCGTGCGGCTC
CGCGCT
GCCCGGCGGCTGTGAGCGCTGCGGGCGCGGCGCGGGGCTTTGTGCGCTCCGCAG
TGTGCG
CGAGGGGAGCGCGGCCGGGGGCGGTGCCCCGCGGTGCGGGGGGGCTGCGAGG
GGAACAA
AGGCTGCGTGCGGGGTGTGTGCGTGGGGGGGTGAGCAGGGGGTGTGGGCGCGTC
 
GGTCGG
GCTGCAACCCCCCCTGCACCCCCCTCCCCGAGTTGCTGAGCACGGCCCGGCTTCG
GGTGC
GGGGCTCCGTACGGGGCGTGGCGCGGGGCTCGCCGTGCCGGGCGGGGGGTGGCG
GCAGGT
GGGGGTGCCGGGCGGGGCGGGGCCGCCTCGGGCCGGGGAGGGCTCGGGGGAGG
GGCGCGG
CGGCCCCCGGAGCGCCGGCGGCTGTCGAGGCGCGGCGAGCCGCAGCCATTGCCT
TTTATG
GTAATCGTGCGAGAGGGCGCAGGGACTTCCTTTGTCCCAAATCTGTGCGGAGCC
GAAATC
TGGGAGGCGCCGCCGCACCCCCTCTAGCGGGCGCGGGGCGAAGCGGTGCGGCGC
CGGCAG
GAAGGAAATGGGCGGGGAGGGCCTTCGTGCGTCGCCGCGCCGCCGTCCCCTTCT
CCCTCT
CCAGCCTCGGGGCTGTCCGCGGGGGGACGGCTGCCTTCGGGGGGGACGGGGCAG
GGCGGG
GTTCGGCTTCTGGCGTGTGACCGGCGGCTCTAGAGCCTCTGCTAACCATGTTCAT
GCCTT
CTTCTTTTTCCTACAGCTCCTGGGCAACGTGCTGGTTATTGTGCTGTCTCATCATT
TTGG
CAAAccggt
SEQ ID NO:3_799~1563(Del12) 
GTCGACATTGATTATTGACTAGTTATTAATAGTAATCAATTACGGGGTCATTAGT
TCATA
GCCCATATATGGAGTTCCGCGTTACATAACTTACGGTAAATGGCCCGCCTGGCTG
ACCGC
CCAACGACCCCCGCCCATTGACGTCAATAATGACGTATGTTCCCATAGTAACGCC
 
AATAG
GGACTTTCCATTGACGTCAATGGGTGGAGTATTTACGGTAAACTGCCCACTTGGC
AGTAC
ATCAAGTGTATCATATGCCAAGTACGCCCCCTATTGACGTCAATGACGGTAAATG
GCCCG
CCTGGCATTATGCCCAGTACATGACCTTATGGGACTTTCCTACTTGGCAGTACAT
CTACG
TATTAGTCATCGCTATTACCATGGTCGAGGTGAGCCCCACGTTCTGCTTCACTCTC
CCCA
TCTCCCCCCCCTCCCCACCCCCAATTTTGTATTTATTTATTTTTTAATTATTTTGTG
CAG
CGATGGGGGCGGGGGGGGGGGGGGCGCGCGCCAGGCGGGGCGGGGCGGGGC
GAGGGGC
GGGGCGGGGCGAGGCGGAGAGGTGCGGCGGCAGCCAATCAGAGCGGCGCGCTC
CGAAAGT
TTCCTTTTATGGCGAGGCGGCGGCGGCGGCGGCCCTATAAAAAGCGAAGCGCGC
GGCGGG
CGGGAGTCGCTGCGCGCTGCCTTCGCCCCGTGCCCCGCTCCGCCGCCGCCTCGCG
CCGCC
CGCCCCGGCTCTGACTGACCGCGTTACTCCCACAGGTGAGCGGGCGGGACGGCC
CTTCTC
CTCCGGGCTGTAATTAGCGGGGGGACGGCTGCCTTCGGGGGGGACGGGGCAGGG
CGGGGT
TCGGCTTCTGGCGTGTGACCGGCGGCTCTAGAGCCTCTGCTAACCATGTTCATGC
CTTCT
TCTTTTTCCTACAGCTCCTGGGCAACGTGCTGGTTATTGTGCTGTCTCATCATTTT
GGCA
AAccggt
SEQ ID NO:4_469~571,799~1563(eCAPE)

GTCGACATTGATTATTGACTAGTTATTAATAGTAATCAATTACGGGGTCATTAGT
TCATA
GCCCATATATGGAGTTCCGCGTTACATAACTTACGGTAAATGGCCCGCCTGGCTG
ACCGC
CCAACGACCCCCGCCCATTGACGTCAATAATGACGTATGTTCCCATAGTAACGCC
AATAG
GGACTTTCCATTGACGTCAATGGGTGGAGTATTTACGGTAAACTGCCCACTTGGC
AGTAC
ATCAAGTGTATCATATGCCAAGTACGCCCCCTATTGACGTCAATGACGGTAAATG
GCCCG
CCTGGCATTATGCCCAGTACATGACCTTATGGGACTTTCCTACTTGGCAGTACAT
CTACG
TATTAGTCATCGCTATTACCATGGTCGAGGTGAGCCCCACGTTCTGCTTCACTCTC
CCCA
TCTCCCCCCCCTCCCCACCCCCAATTTTGTATTTATTTATTTTTTAATAGCCAATCA
GAG
CGGCGCGCTCCGAAAGTTTCCTTTTATGGCGAGGCGGCGGCGGCGGCCCTAT
AAAAA
GCGAAGCGCGCGGCGGGCGGGAGTCGCTGCGCGCTGCCTTCGCCCCGTGCCCCG
CTCCGC
CGCCGCCTCGCGCCGCCCGCCCCGGCTCTGACTGACCGCGTTACTCCCACAGGTG
AGCGG
GCGGGACGGCCCTTCTCCTCCGGGCTGTAATTAGCGGGGGGACGGCTGCCTTCGG
GGGGG
ACGGGGCAGGGCGGGGTTCGGCTTCTGGCGTGTGACCGGCGGCTCTAGAGCCTC
TGCTAA
CCATGTTCATGCCTTCTTCTTTTTCCTACAGCTCCTGGGCAACGTGCTGGTTATTG

TGCT
GTCTCATCATTTTGGCAAAccggt

## Claims

1. An isolated polynucleotide consisting of a nucleotide sequence in which at least the region from base 469 to base 571 is deleted from a nucleotide sequence described in SEQ ID NO: 1, or consisting of a nucleotide sequence having 90% or more identity thereto.

2. The polynucleotide according to claim 1, consisting of a nucleotide sequence described in SEQ ID NO: 2, or consisting of a nucleotide sequence having 90% or more identity thereto.

3. An isolated polynucleotide consisting of a nucleotide sequence in which at least the region from base 799 to base 1563 is deleted from a nucleotide sequence described in SEQ ID NO: 1, or consisting of a nucleotide sequence having 90% or more identity thereto.

4. The polynucleotide according to claim 3, consisting of a nucleotide sequence described in SEQ ID NO: 3, or consisting of a nucleotide sequence having 90% or more identity thereto.

5. An isolated polynucleotide consisting of a sequence in which at least the following regions (a) and (b) are deleted from a nucleotide sequence described in SEQ ID NO: 1, or consisting of a nucleotide sequence having 90% or more identity thereto:
(a) the region from base 469 to base 571; and
(b) the region from base 799 to base 1563.

6. The polynucleotide according to claim 5, consisting of a nucleotide sequence described in SEQ ID NO: 4, or consisting of a nucleotide sequence having 90% or more identity thereto.

7. A promoter consisting of the polynucleotide according to any one of claims 1 to 6.

8. A vector comprising the promoter according to claim 7.

9. A vector comprising the promoter according to claim 7 and a gene encoding a protein.

10. A transformed cell obtained by transforming a mammalian cell with the vector according to claim 9.

11. The transformed cell according to claim 10, wherein the mammalian cell is a CHO cell.

12. A method for producing a recombinant protein, comprising a step of culturing the transformed cell according to claim 11 to produce a recombinant protein, and a step of recovering the produced recombinant protein from the resulting culture.

13. The method according to claim 12, which is a method for producing an antibody, wherein the vector comprises a promoter consisting of a nucleotide sequence described in any one of SEQ ID NOs: 2, 3, or 4, and a gene encoding an antibody, the gene encoding an amino acid sequence comprising a VL or a VH of the antibody, or encoding amino acid sequences comprising the VL and VH of the antibody.

14. An isolated polynucleotide fragment having a promoter function, consisting of a sequence having at least 70%, at least 80%, at least 90%, or 100% identity to the sequence described in any one of SEQ ID NOs: 2, 3, or 4.

15. The vector according to claim 9, wherein the protein is a heavy chain (H chain) or a light chain (L chain) of an antibody.

16. A cell comprising a gene into which a promoter consisting of a nucleotide sequence described in any one of SEQ ID NOs: 2, 3, or 4 is incorporated.

17. The cell according to claim 16, wherein the gene encodes an antibody polypeptide.

18. A method for producing an antibody using a cell comprising a gene into which a promoter consisting of a nucleotide sequence described in any one of SEQ ID NOs: 2, 3, or 4 is incorporated.
